# EUROPEAN PATENT SPECIFICATION

(11) **EP 1 931 287 B1**
(45) Date of publication and mention of the grant of the patent: **18.12.2013**
(21) Application number: 06825438.2
(22) Date of filing: 05.10.2006
(51) Int. Cl.: A61F 5/04, A61B 17/70

(54) **BONE ALIGNMENT IMPLANT**
KNOCHENAUSRICHTUNGSIMPLANTAT
IMPLANT POUR ALIGNEMENT OSSEUX

(30) Priority: 06.10.2005 US 244879
(43) Date of publication of application: 18.06.2008
(60) Divisional application: 13190892.3
(73) Proprietor: AMEI TECHNOLOGIES Inc., Wilmington, Delaware 19899 (US)
(72) Inventor: STEVENS, Peter, M., Salt Lake City, UT 84113 (US); REINECKE, Steven, M., Dallas, TX 75205 (US); VASTA, Paul, J., McKinney, TX 75069 (US); THOMAS, Michael, Gregory, Van Alstyne, TX 75495 (US)
(74) Representative: Lawrence, John
(86) International application number: PCT/US2006/038773
(87) International publication number: WO 2007/044391

(56) References cited:
- EP-A1- 1 568 329
- WO-A2-2005/032358
- US-A- 5 360 450
- US-A1- 2002 198 527
- US-A1- 2004 210 218
- US-A1- 2005 085 815
- US-A1- 2005 149 023
- US-A1- 2005 192 577

## Description

### FIELD OF THE INVENTION

The present invention relates to the design for an implant to help realign spinal angular and rotational deformities, used particularly in a method for correcting spinal deformities in patients with active growth plates.

### BACKGROUND OF THE INVENTION

As a result of congenital deformation, traumatic injury or other causes, long bones such as the femur, tibia and humerus may grow out of alignment, causing deformity of the limb and biomechanical abnormalities. While some deformities are asymptomatic or may resolve spontaneously, it is often necessary to intervene surgically to realign these limbs. For the patients requiring surgical intervention, both osteotomy with realignment of the bone and epiphyseal stapling are currently accepted methods of treatment.

One common method of surgical bone realignment is by means of an osteotomy, or cutting of the bone, followed by realignment of the bone. In some procedures the bone is cut laterally, transverse to the longitudinal axis of the bone. Then the bone is realigned. A bone graft is then placed in the resulting wedge space. The bone and the bone graft are stabilized by orthopedic fragment fixation implants such as screws and bone plates. In an alternative osteotomy procedure, a bone wedge is removed. The bone is realigned, and similar implants are used to secure the bone. A third method of deformity correction via osteotomy is to first cut the bone, then apply an external frame attached to pins drilled through the skin and into the bone. By adjusting the frame, either intraoperatively or postoperatively, the bone is straightened.

Because osteotomy methods require a relatively large incision to create bone cuts, they are relatively invasive; they disrupt the adjacent musculature and may pose a risk to the neurovascular structures. An additional disadvantage of these procedures is the potential risk of damage to the growth plate, resulting in the disruption of healthy limb growth. Consequently, this procedure may be reserved for bone alignment in skeletally mature patients in whom the growth plates are no longer active.

One less invasive method of bone aligmnent involves the placement of constraining implants such as staples around the growth plate of the bone to restrict bone growth at the implant site and allow the bone to grow on the opposite side. First conceived in 1945 by Dr. Walter Blount, this method is known as epiphyseal stapling. Typically epiphyseal stapling is more applicable in young pediatric patients and adolescents with active growth plates. A staple is placed on the convex side of an angular deformity. Since the bone is free to grow on the concave side of the deformity, the bone tends to grow on the unstapled side, causing the bone to realign over time. Once the bone is aligned, the constraining implants are typically removed.

As long as the growth plate is not disturbed, this type of intervention is generally successful. However, the procedure must be done during the time that the bone is still growing, and the physiodynamics of the physis (growth plate) must not be disturbed. With proper preoperative planning and placement of the implants, the surgeon can use the implants to slowly guide the bone back into alignment.

The implants currently used in epiphyseal stapling procedures are generally U-shaped, rigid staples. The general design has essentially remained the same as those devised by Blount in the 1940's. Since these implants are rigid, they act as three-dimensional constraints prohibiting expansion of the growth plate. They are not designed to allow flexibility or rotation of the stapled legs with the bone sections as the bone is realigned. Due to the constraints of these staple implants, the planning associated with the placement of the implants is overly complicated. Consequently, the surgeon must not only determine where to position the implant across the physis, but also must account for the added variables of implant stiffness, implant strength and bone-implant interface rupture.

The force associated with bone growth causes bending of these implants proportionate to their stiffness. Depending on the strength of the implant, these loads could eventually cause the implants to fracture under the force of bone realignment. This can make them difficult or impossible to remove. These same forces can also cause the implants to deform, weakening the bone-to-implant interface. This weakening may result in migration of the implant out of the bone, risking damage to the adjacent soft tissues and failure of the procedure.

Spinal deformities, including for example scoliosis, which is a lateral deviation from the normal of the spine, arise congenitally, ideopathically, or may result from neuromuscular weakness or paralysis. Some cases of scoliosis, generally those having less than about 20 degrees curvature, need only be observed to watch for progression. More severe cases may require treatment, with treatment options ranging from bracing to surgery. Scoliosis surgery has, until recently, involved fusing the vertebrae in the curved area together, thereby conecting the curvature.

Scoliosis usually appears in children or teenagers. The use of braces in such patients may be emotionally difficult, negatively impacting self-image and self-esteem. Surgical treatment options entail risks of spinal cord or nerve damage, failure of the bones to fuse, and spine infection. Moreover, successful bone fusion results in impaired spinal motion which could limit or prevent certain physical activities. Impaired mobility appears to have a particularly negative impact in children and young adults as it generally prevents their participation in sports and social activities. In addition, the impaired mobility concomitant with spinal fusion increases the likelihood of back pain as the patient ages.

More recently, the use of staples has been tested in scoliosis treatment. More specifically, intervertebral body stapling on the convex side of the anterior spine while permitting continued and unrestricted growth on the concave side of the curve has been tested. Such procedure has shown some level of effectiveness in patients with active growth plates. In adult patients without or with insufficient growth plate activity, stapling in conjunction with wedge osteotomies have been used to correct spinal curves.

Again, as discussed above in connection with long bones, as long as the growth plate is not disturbed, this type of intervention is generally successful. However, the procedure must be done during the time that the bone is still growing, and the physiodynamics of the physis (growth plate) of the tethered vertebrae must not be disturbed. With proper preoperative planning and placement of the implants, the surgeon can use the implants to slow growth on the convex side of the spinal deformity while allowing growth on the concave side of the deformity thereby bringing the spinal column into alignment.

The force associated with bone growth causes bending of implants such as rigid staples with the bending proportionate to their stiffness. Depending on the strength of the implant, these loads could eventually cause the implants to fracture. This can make them difficult or impossible to remove. These same forces can also cause the implants to deform, weakening the vertebra-to- implant interface. This weakening may result in migration of the implant out of the vertebra, risking damage to the adjacent soft tissues. Such risk is particularly high in the spinal column where damage to adjacent tissues could result in permanent disability or worse, or depending on the location of the staple and its migration.

To prevent staple migration, pronged staples have been tested in correction of spinal deformities. Such staples, however, are not useful in correction of spinal rotation. That is, pronged staples are limited to use in cases of two-dimensional curvature; anterior-posterior, medial-lateral, and cranial-caudal.

US2005/0085815 discloses a rod-shaped implant element for the connection of bone anchoring elements, each bone anchoring element having an anchoring section to be anchored in the bone and a receiver member to be connected to the rod-shaped implant element, one embodiment having a tee rod comprising rigid sections dimensioned such that three pedicle screws can be connected thereto, a flexible section provided adjacent thereto and between the pedicle screws, and another rigid section provided to be connected to one of the pedicle screws.

### SUMMARY OF THE INVENTION

The present description relates to an orthopedic bone alignment implant system that includes a guide wire, a link and bone fasteners. The guide wire serves to locate the growth plate under fluoroscopic guidance. The bone fasteners and the link function together as a tether between bone segments on opposite sides of the physis. As the bone physis generates new physeal tissue, the bone alignment implant tethers between engagers on the bone segments. This tethering principle guides the alignment of the bone as it grows.

Although applicable in various orthopedic procedures, involving fracture fixation, the bone alignment implant is also applicable to the correction of angular deformities in long bones in which the physis is still active.

The distal end of the guide wire is used to locate the physis. Once its tip is placed in the physis, it is driven partly into the physis to function as a temporary guide for the link. The delivery of the implant over the guide wire assures that the link is properly placed with the bone fasteners on opposite sides of the physis. This will minimize the chance of damaging the physis throughout bone realignment. The link is then placed over the guide wire and oriented such that openings through the link for the bone fasteners are on either side of the physis. For pure angular correction, these openings would be collinear with the long axis of the bone; for rotational correction, they would be oblique to its axis.

The bone fasteners are then placed through the openings in the link and into the bone, connecting the sections of bone on opposite sides of the physis with the implant. Alternatively, guide pins can be used to help align cannulated fasteners.

The implant is designed such that it partially constrains the volume of the bone growth on the side of the physis that it is placed. The implant guides the growth of new bone at the physis such that the growth direction and resulting alignment is controlled. The implant limits the semi- longitudinal translation of the bone fasteners yet allows for the bone fasteners to freely rotate with the bone segments as the angular or torsional deformity is straightened.

In accordance with the invention, a bone alignment implant for correcting spinal deformities is disclosed in claim 1.

In some embodiments of the bone alignment implant system the link of each bone alignment implant is adapted to span the between the one or more intervertebral spaces.

In other embodiments of the bone alignment implant system the link of one or more bone alignment implant is a flexible link, whereas, in other embodiments, the link of one or more bone alignment implant is a rigid link.

In some embodiments of the bone alignment implant system, the first engager of one or more bone alignment implant is a threaded engager. In other embodiments of the bone alignment implant system, the second engager of one or more bone alignment implant is a threaded engager. In yet other embodiments, both the first and second engagers are threaded.

In some aspects of the bone alignment implant system, the first fastener of one or more bone alignment implant further has a first head, and a first shaft, the second fastener of one or more bone alignment implant further has a second head and a second shaft. In some embodiments, the first shaft of one or more bone alignment implant is a deformable shaft. In other embodiments, the second shaft of one or more bone alignment implant is a deformable shaft. In yet other embodiments, both the first and second shafts are deformable.

In some aspects of the bone alignment implant system, the first engager of one or more bone alignment implant is a barbed engager. In other aspects of the bone alignment implant system, the second engager of one or more bone alignment implant is a barbed engager. In yet other embodiments of the bone alignment implant system, both the first and second engagers are barbed engagers.

Also described herein is a method of correcting a spinal deformity wherein such method includes the steps of: (1) implanting a bone alignment implant intervertebrally on a convex side of a spinal deformity with a first bone engager attached to a first vertebral body of a first vertebra and a second bone engager attached to a second vertebral body of a second vertebra; and (2) tethering the first vertebra to the second vertebra on the convex side of the deformity with the bone alignment implant.

In some examples of the method of correcting a spinal deformity, the method further includes the step of: (3) allowing the physis of the first vertebra and the physis of the second vertebra to generate more tissue on the concave side of the spinal deformity than on the convex side of the spinal deformity.

In some aspects of the method of correcting a spinal deformity, the bone alignment implant is attached substantially vertically along the length of a spine.

In some aspects of the method of correcting a spinal deformity the first bone engager is axially displaced from the second bone engager.

In some aspects of the method of correcting a spinal deformity the first and second vertebra are adjacent.

In some aspects of the method of correcting a spinal deformity the first vertebra is located in the cervical spine, the thoracic spine, or the lumbar spine.

In some aspects of the method of correcting a spinal deformity the spinal deformity is a two-dimensional curvature selected from the group of anterior-posterior, medial-lateral, and cranial-caudal curvatures.

In some aspects of the method of correcting a spinal deformity the first and second bone engagers are allowed to rotate with respect to the bone alignment implant.

In some aspects of the method of correcting a spinal deformity the spinal deformity is a three-dimensional curvature, including spinal rotation.

In some aspects of the method of correcting a spinal deformity, the method further includes the step of performing a wedge osteotomy on the first vertebra on the concave side of the deformity.

In some aspects of the method of correcting a spinal deformity, the method further includes the step of performing a wedge osteotomy on the first vertebra distal from the bone alignment implant.

In some examples of the method of correcting a spinal deformity, the bone alignment implant comprises a first fastener with a first engager adapted for fixation with bone; a second fastener with a second engager adapted for fixation with bone; and a link connecting the first fastener to the second fastener.

In some examples of the method of correcting a spinal deformity the link is expandable to provide varying distances between the first engager and the second engager.

In some aspects of the method of correcting a spinal deformity the link is retractable to provide varying distances between the first engager and the second engager.

In some aspects of the method of correcting a spinal deformity the link is a flexible link. In other embodiments of the method of correcting a spinal deformity, the link is a rigid link.

In some aspects of the method of correcting a spinal deformity,

the link is made from a memory metal. In some aspects of the method of correcting a spinal deformity the memory force of the link exerts a force selected from the group of a force restricting growth, a force assisting growth, and a rotational force.

In some aspects of the method of correcting a spinal deformity the first engager is a threaded engager. In some aspects of the method of correcting a spinal deformity the second engager is a threaded engager. In yet other aspect of the method of correcting a spinal deformity, both the first and second engagers are threaded.

In some aspects of the method of correcting a spinal deformity the first fastener further has a first head, and a first shaft, the second fastener further has a second head and a second shaft. In certain aspects of the method of correcting a spinal deformity, the first shaft is a deformable shaft, in other aspects of the method of correcting a spinal deformity, the second shaft is a deformable shaft, in yet other aspects of the method, both the first and second shafts are deformable.

In some aspects of the method of correcting a spinal deformity the first engager is a barbed engager. In yet other aspects of the method of correcting a spinal deformity, the second engager is a barbed engager. In yet other aspects of the method of correcting a spinal deformity, both the first and second engagers are barbed.

In some aspects of the method of correcting a spinal deformity, the method further includes a first joint between the first fastener and the link; a second joint between the second fastener and the link; wherein the first joint allows tilt between the first fastener and the link, and limits longitudinal displacement and axial displacement between the first fastener and the link; and the second joint allows tilt between the second fastener and the link, and limits longitudinal displacement and axial displacement between the second fastener and the link; wherein the bone alignment implant functions as a tether between the first bone engager and the second bone engager.

Also described is a method of correcting a spinal deformity including the steps of: (1) implanting a bone alignment implant intravertebrally on a convex side of a spinal deformity with a first bone engager attached to a diaphyseal section of a vertebral body of a vertebra and a second bone engager attached to a metaphyseal section of the vertebral body of the vertebra; (2) allowing the physis of the vertebral body of the vertebral body of the vertebra to generate new physeal tissue; (3) tethering the diaphyseal section of the vertebral body of the first vertebra to the metaphyseal section of the vertebral body of the vertebra on the convex side of the deformity with the bone alignment implant; and (4) allowing the physis of the vertebral body of the vertebra to generate more tissue on the concave side of the spinal deformity than on the convex side of the spinal deformity.

Other examples provide a method of implanting a bone alignment implant in a spinal column including the steps of: (1) locating the center of a first vertebral body of a first vertebra; (2) locating the center of a second vertebral body of a second vertebra; (3) attaching a bone alignment implant across the first and second vertebra comprising the steps of: (a) passing a first bone engager through a first opening in the bone alignment implant and attaching the first bone engager to the first vertebral body at about the center of the first vertebral body; and (b) passing a second bone engager through a second opening in the bone alignment implant and attaching the second bone engager to the second vertebral body at about the center of the second vertebral body.

In some examples of the in method of implanting a bone alignment implant in a spinal column, the first vertebral body is located using a guidewire passed through the first opening.

### BRIEF DESCRIPTION OF THE DRAWINGS

Various embodiments of the present invention will now be discussed with reference to the appended drawings. It is appreciated that these drawings depict only typical embodiments of the invention and are therefore not to be considered limiting of its scope.

FIG. 1 is an anterior view of the knee showing a genu valgum deformity (knee knocking) in the femur and the insertion of a guide wire approximately parallel to the physis;

FIG. 2 is a sagittal view of that described in FIG. 1 showing the placement of the guide wire in the physis;

FIG. 3 is anterior view of the knee showing the placement of a link and drill guide over the guide wire and the use of the guide to place two guide pins for fasteners on opposite sides of the physis;

FIG. 4 is a sagittal view of the placement of the link described in FIG. 3 showing the position of the two guide pins on opposite sides of the physis;

FIG. 5 is an alternative method of applying the link over the guide wire in which the link is placed first, then the fasteners are placed through the openings in the link;

FIG. 6 is a sagittal view of the link placement also shown in FIG. 5;

FIG. 7 is an anterior view showing an alternative method of drilling of holes in the bone over the guide pins to prepare the bone for the fasteners;

FIG. 8 is a anterior view of the link showing the placement of the fasteners through the link and into the bone segments;

FIG. 9 is a sagittal view of the fasteners and link described in FIG. 8;

FIG. 10 is an anterior view as seen after the physeal tissue has grown and the bone alignment implant assembly has been reoriented as the bone is realigned;

FIG. 11 is sagittal view of the bone alignment implant placed on a rotational deformity;

FIG. 12 is the same sagittal view described in FIG. 11 after the rotational deformity has been corrected;

FIG. 13 is a perspective view of a threaded fastener;

FIG. 14 is perspective view of a barbed fastener;

FIG. 15 is perspective view of an alternative example of the bone alignment implant with rigid link and fasteners, with joints allowing restricted movement between them;

FIG. 16 is a perspective view of an alternative example of the bone alignment implant showing a flexible midsection of the link with rigid material surrounding the openings;

FIG. 17 is a perspective view of an alternative example of the bone alignment implant showing a flexible midsection of the link made from a separate flexible member with rigid material surrounding the openings;

FIG. 18 is a perspective view of an alternative example of the bone alignment implant showing flexible woven material throughout the body of the link with reinforcement grommets surrounding the openings;

FIG. 19 is a perspective view of an alternative example of the bone alignment implant showing the link made from a flexible band of material;

FIG. 20 is a perspective view of an alternative example of the bone alignment implant showing the link made from a flexible ring of braided material that is joined in the midsection, forming two openings;

FIG. 21 is a side view of an alternative example of the bone alignment implant showing a bone fasteners that have flexible shaft sections;

FIG. 22 is a side view of an alternative example of the bone alignment implant showing two barbed bone fasteners attached to a flexible link; and

FIG. 23 is a side view of an alternative example of the bone alignment implant showing one barbed bone fastener and one threaded bone fastener connected to a flexible link.

FIG. 24 is an anterior view of the bone alignment implant of FIG. 15 attached to vertebral bodies on the convex side of a spinal curvature.

FIG. 25 is a sagittal view of the bone alignment implant of FIG. 15 attached to vertebral bodies on the convex side of a spinal curvature.

FIG 26 is a sagittal view of an alternative example of the bone alignment implant as implanted to correct a rotational deformity.

FIG. 27 is a sagittal view of the implant shown in FIG. 26 showing correction of a rotational deformity following vertebra growth.

FIG.28 is a sagittal view showing use of three bone alignment implants in a "chain" structure attached to vertebral bodies on the convex side of a spinal curvature.

### DESCRIPTION OF EMBODIMENTS OF THE INVENTION

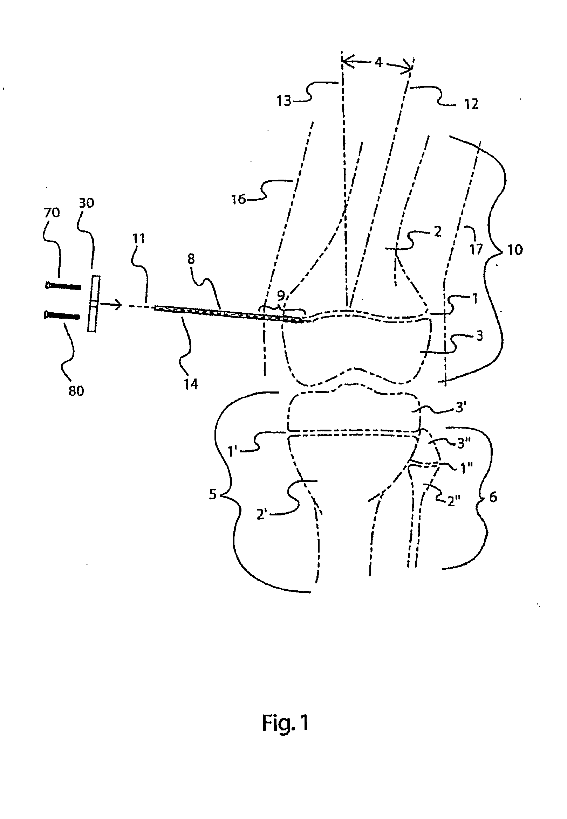

Referring to FIG. 1, a schematic anterior view of the human knee joint is depicted in which a distal femur 10 is proximal to a proximal tibia 5 and a proximal fibula 6. A distal femoral physis 1, or growth plate, separates a distal metaphyseal section 3 from a proximal diaphyseal section 2 of the distal femur 10. Likewise a proximal tibial physis 1' separates a proximal metaphyseal section 3' from a diaphyseal section 2' of the proximal tibia 5 and a proximal fibula physis 1" separates a proximal metaphyseal section 3" of a proximal fibula 6 from a diaphyseal section 2" of the proximal fibula 6. Although the implant described herein is adaptable to nearly all of the long bones in the body, only the example of correcting one type of an angular deformity in the distal femur will be described in detail. The principles described herein can be adapted to other deformities and other bones such as the tibia, fibula, humerus, radius and ulna.

By example, an angular deformity 4 in the femur 10 known as genu valgum or knock- knee is shown in FIG. 1. The angular deformity 4 is the angle between a pretreatment longitudinal axis 12 of the femur 10 and a post treatment projected longitudinal axis 13 of the femur 10. A bone alignment implant will be placed on the medial side of the femur 10. In this case, the medial side of the femur 10 is curved in a convex arc. Hence, this side of the deformity is called a convex side 16 because the angular deformity 4 bends the femur 10 in a curve that is angled away from or convex with respect to the medial side. A concave side 17 is on the opposite side of the femur 10. Likewise, the angular deformity 4 is angled towards the concave side 17.

A guide wire 8, as shown in FIG. 1, is used to locate the physis and guide the bone alignment implant to the surgical site. The guide wire 8 comprises a long axis 11, a distal section 9 that is shaped to fit into the physeal tissue, and a periphery 14 that is typically a constant size and shape. In this case, the shape of the guide wire 8 along the long axis 11 is essentially cylindrical so the shape of the periphery 14 is round and does not change except for in the distal section 9. However, the periphery 14 can be a variable cross-section that changes shape or size along the length of the long axis 11.

In this example, the long axis 11 of the guide wire 8 is placed into and approximately parallel with the physis 1 and is aligned approximately in the same plane as the angular deformity 4. As shown in FIG. 1, the distal section 9 of the guide wire 8 is partly inserted into the physis 1. Since the cartilaginous physis 1 is of less density than the surrounding bone, the surgeon can either poke the distal section of the guide wire 8 into the bone until the physis 1 is located, or the surgeon can use fluoroscopic x-ray (not shown) or other bone density detection means (not shown) to determine the location of the physis 1 relative to the distal section of the guide wire 8 to place the guide wire 8 in a direction that is approximately parallel with the physis 1.

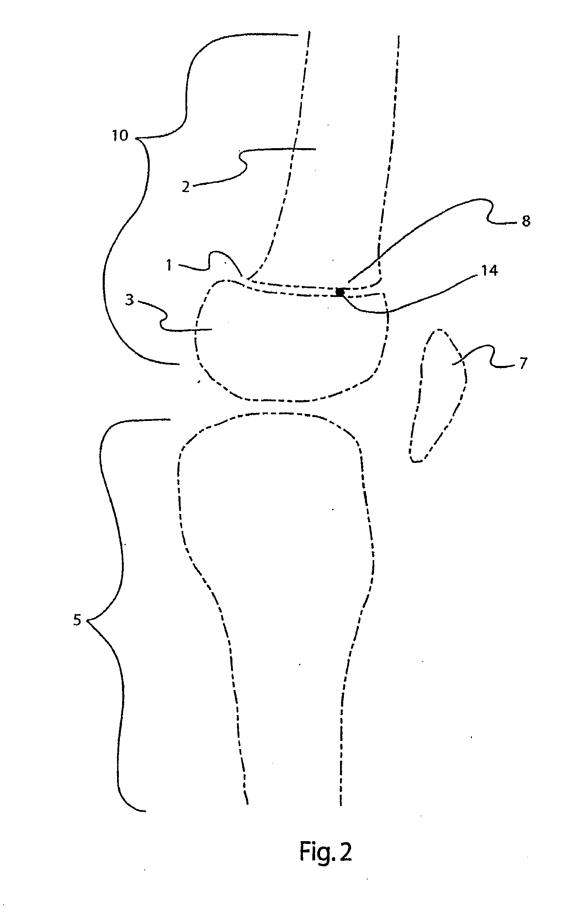

FIG. 2 is a sagittal view approximately perpendicular to the anterior view described in FIG. 1. For reference, a patella 7 is shown on the anterior side of the femur 10 and tibia 5. For clarity, in this example the guide wire 8 is straight and has a constant round outer periphery 14. Consequently, only the outer periphery 14 of the guide wire 8 is shown and appears as a circle in FIG. 2. FIG. 2 shows the placement of the guide wire 8 in the physis between the femoral diaphyseal section 2 and the distal femoral metaphyseal section 3. This is the preferred placement of the guide wire 8. The guidewire 8 is used to locate an area in the physis that will eventually be bridged by the bone alignment implant 9 that will tether between two sections of the bone. In FIG. 2, the two sections of bone that will be tethered by the bone alignment implant 9 are the distal femoral proximal metaphyseal section 3 and the femoral diaphyseal section 2.

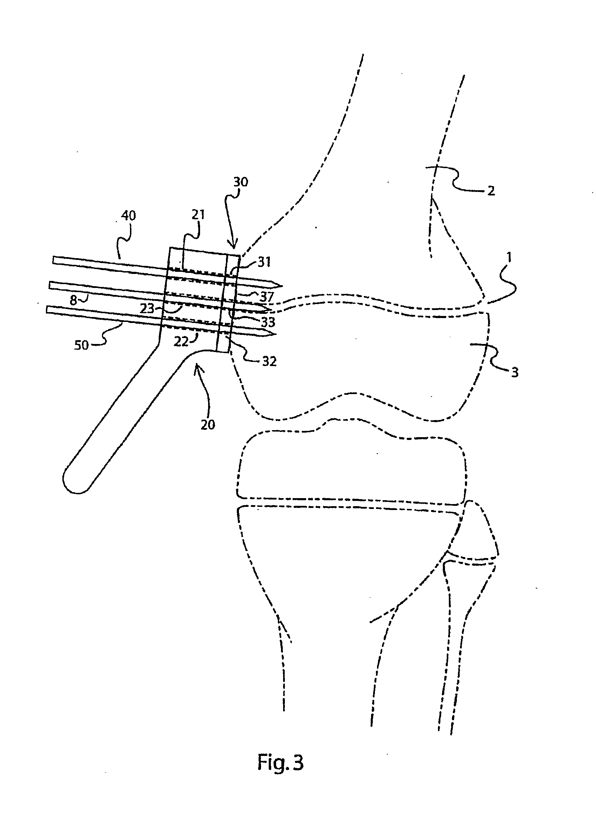

FIG. 3 is an anterior view of the knee showing the placement of a link 30 and a guide 20 over the guide wire 8. The guide 20 is used to place a first guide pin 40 and a second guide pin 50 on opposite sides of the physis. The link 30 has an outer periphery 34 that defines the outer material bounds of the link 30, a bone side 37 that is the side of the link that is placed against the bone, a first opening 31 and a second opening 32.

First, the guide 20 and link 30 are placed over the guide wire 8 by guiding the guide wire 8 over a guide opening 33 in the link 30 and the guide hole 23 in the guide 20. Then the first guide pin 40 is driven through a first hole 21 in the guide 20 and through the first opening 31 in the link 30 into the diaphyseal bone 2, and the second guide pin 50 is driven through a second hole 22 in the guide 20 and the second opening 32 in the link 30 into the distal metaphyseal section 3. Once the first guide pin 40 and the second guide pin 50 are placed, the guide 20 is removed.

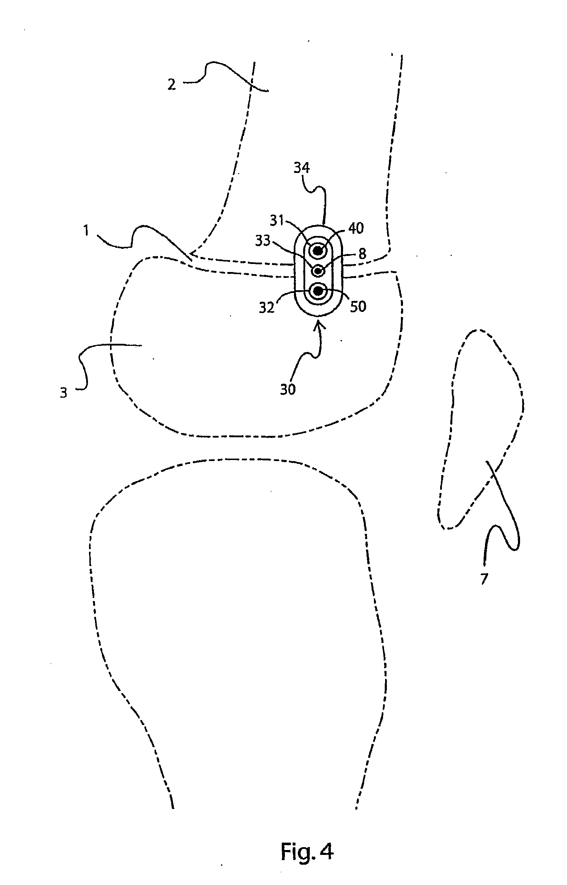

FIG. 4 is a sagittal view of the placement of the link 30 described in FIG. 3. The position of the first guide pin 40 is through the first opening 31 in the link 30. The position of the second guide pin 50 is though the second opening 32 in the link 30. The guide pin 40 and guide pin 50 are on opposite sides of physis 1. Likewise, the first opening 31 and the second opening 32 are on opposite sides of the physis 1.

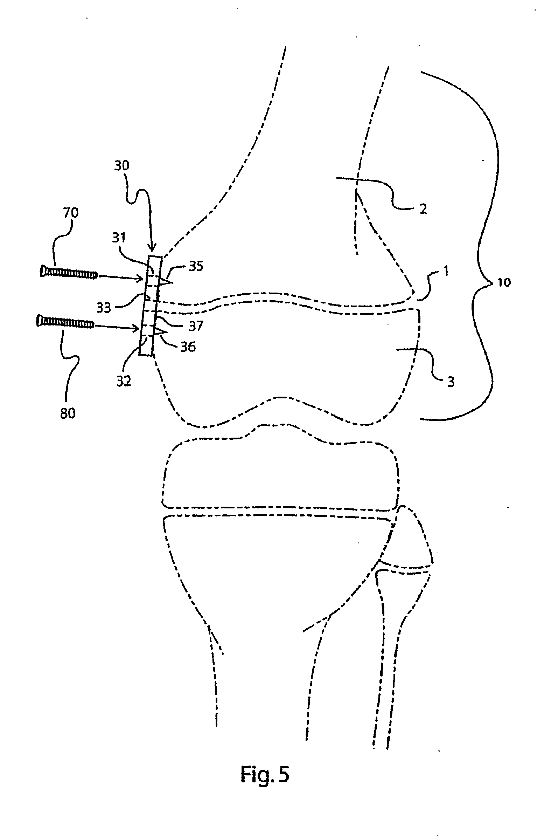

FIG. 5 is an anterior view showing an alternative example of the link 30 placed on the medial femur 10. In this embodiment, a first set of spikes 35 and a second set of spikes 36 on the bone side 37 of the link 30 help to keep the link 30 in place prior to the placement of a first bone fastener 70 and a second bone fastener 80. The first set of spikes 35 is positioned near the first opening 31 and the second set of spikes 36 is positioned near the second opening 32 in the link 30. Hence, as the link 30 is placed across the physis 1, the first set of spikes 35 contacts the diaphyseal section 2 and the second set of spikes 36 contacts the metaphyseal section 3. In this embodiment, the first bone fastener 70 is placed though the first opening 31 in the link 30 then into the diaphyseal section 2 and the second bone fastener 80 is placed through the second opening 32 in the link 30 then into the metaphyseal section 3.

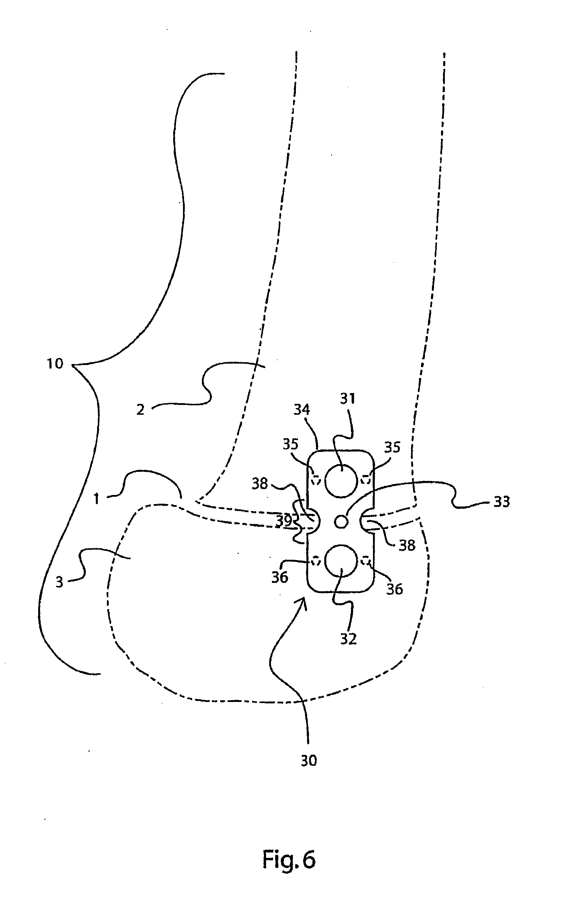

FIG. 6 is a sagittal view of the link 30 on the femur 10 showing the location of the first set of spikes 35 near the first opening 31 on the diaphyseal section 2 side of the physis 1 and the location of the second set of spikes near the second opening 32 on the metaphyseal side of the physis 1.

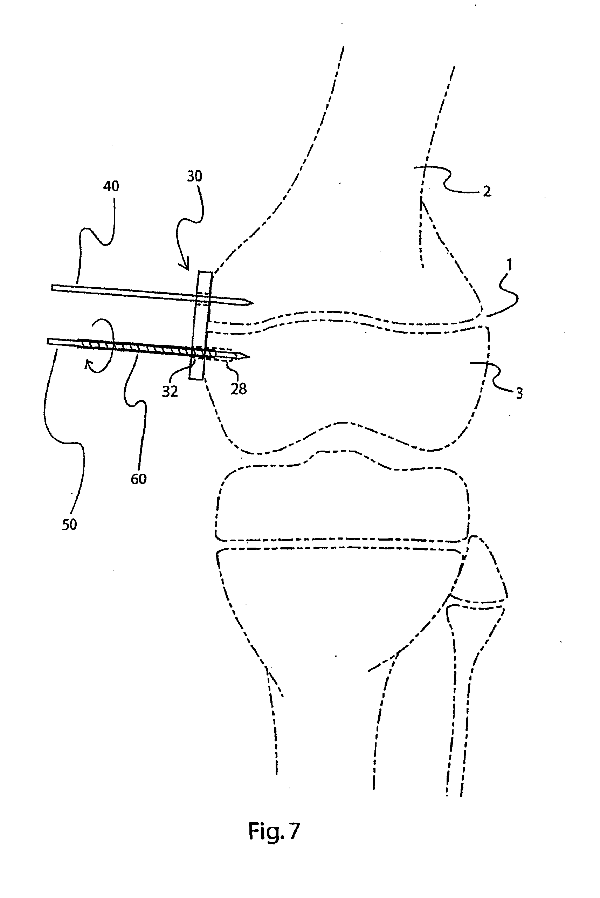

FIG. 7 is an anterior view of the placement of the link 30, first guide pin 40, and second guide pin 50 as previously described in the sagittal view shown in FIG. 4. FIG. 7 also shows a bone preparation tool 60 that can be used to prepare a bore 28 in the bone prior to the first fastener 70 or second fastener 80 placements. The bone preparation tool 60 can be a drill, tap, rasp, reamer, awl or any tool used to prepare a bore in bone tissue for a fastener. The bone preparation tool 60 is used to prepare a bore 28 the bone near the second opening in the metaphyseal section 3 for the second fastener 80. A bone preparation tool 60 can also be used to prepare the bone in the diaphyseal section 2 for the first fastener 70. In the case of the example shown in FIG. 7, the bone preparation tool 60 is placed over the second guide pin 50, through the second opening 32, and into the metaphyseal section 3. However, the bone preparation tool 60 can also be placed directly through the second opening 32 without the guidance of the second guide pin 50. The bone preparation tool 60 is used if needed to prepare the bone to receive the first fastener 70 and second fastener 80. Once the bone is prepared, the bone preparation tool 60 is removed from the surgical site.

The first fastener 70 is then placed over the first guide pin 40, through the first opening 31, and into the diaphyseal section 2. The second fastener 80 is placed over the second guide pin 50, through the second opening 32 and into the metaphyseal section 3. If the first guide pin 40 and second guide pin 50 are not used, the first fastener 70 is simply driven through the first opening 31 and the second fastener 80 is simply driven though the second opening 32 without the aid of the guide pins 40 and 50.

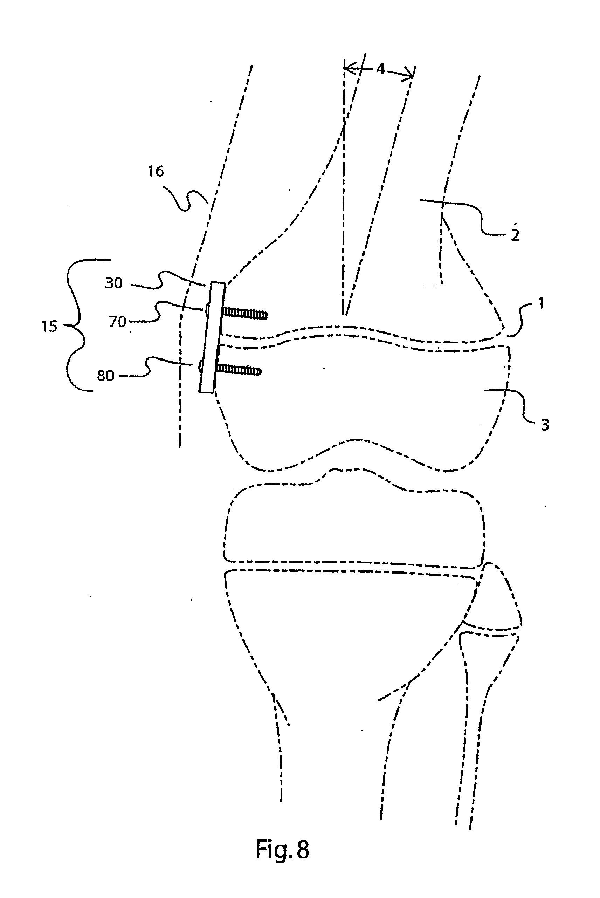

FIG. 8 is an anterior view showing the position of a bone alignment implant 15 on the convex side 16 of the angular deformity 4. The bone alignment implant 15 comprises the link 30, the first fastener 70, and the second fastener 80. The bone alignment implant 15 functions as a tether connecting the diaphyseal section 2 and the metaphyseal section 3. The first fastener 70 and the second fastener 80 are placed on opposite sides of the physis 1. As the physis 1 generates new physeal tissue 90, the physeal tissue 90 will fill in between the diaphyseal section 2 and the metaphyseal section 3 in the space subjected to the least resistance. The bone alignment implant 15 restricts the longitudinal movement between the metaphyseal section 3 and the diaphyseal section 3 on the convex side 16 of the angular deformity 4.

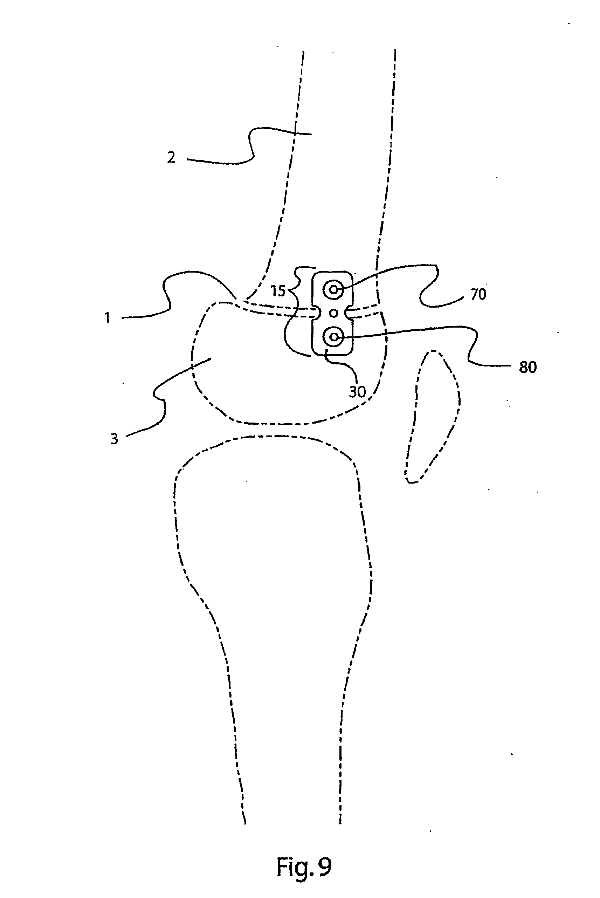

FIG. 9 shows the sagittal view of that described for FIG. 8. The bone alignment implant 15 functioning as a tether restricting the longitudinal movement between the metaphyseal section 3 and the diaphyseal section 2.

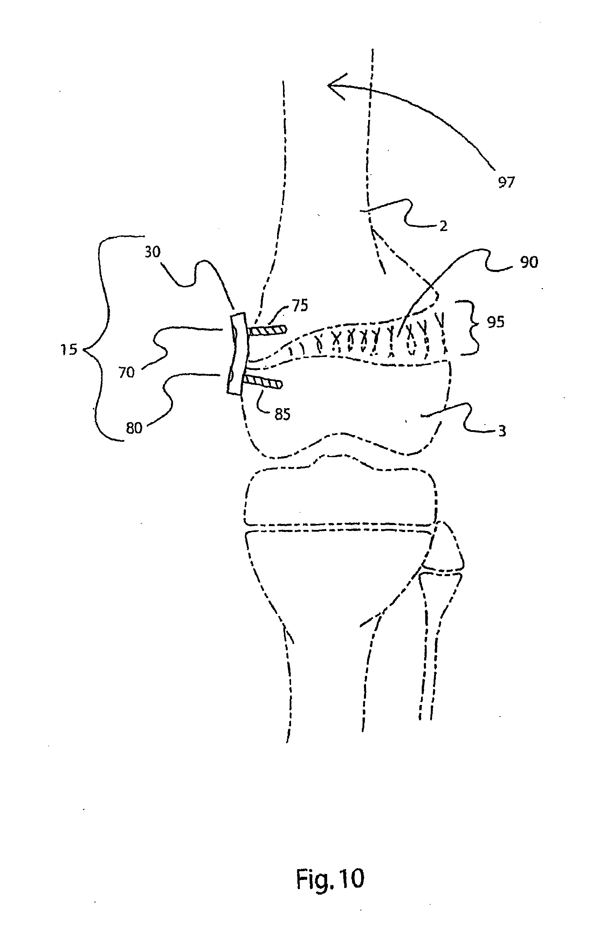

As shown in FIG. 10, in a patient with an active physis, the newly generated physeal tissue 90 fills in more on the side of the bone that is not tethered by the bone alignment implant 15. Hence, a net gain 95 of physeal tissue 90 forces the bone to align in the direction of an angular correction 97.

Select examples of the bone alignment implant 15 comprise the first fastener 70 having a first engager 75, the second fastener 80 having a second engager 85 and the link 30. The link 30, the first fastener 70 and the second fastener 80 function together as tethers between a first engager 75 on the first fastener 70 and a second engager 85 on the second fastener 80, guiding movement between the metaphyseal section 3 and diaphyseal section 2 of bone.

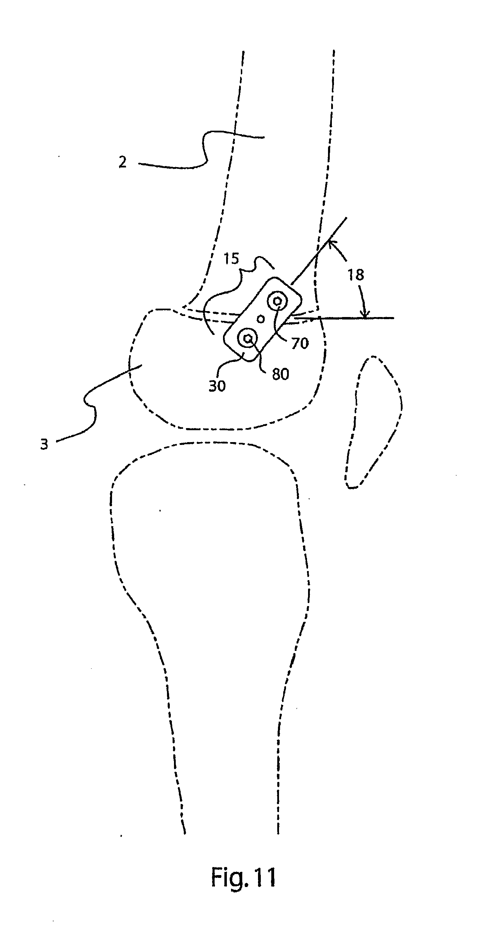

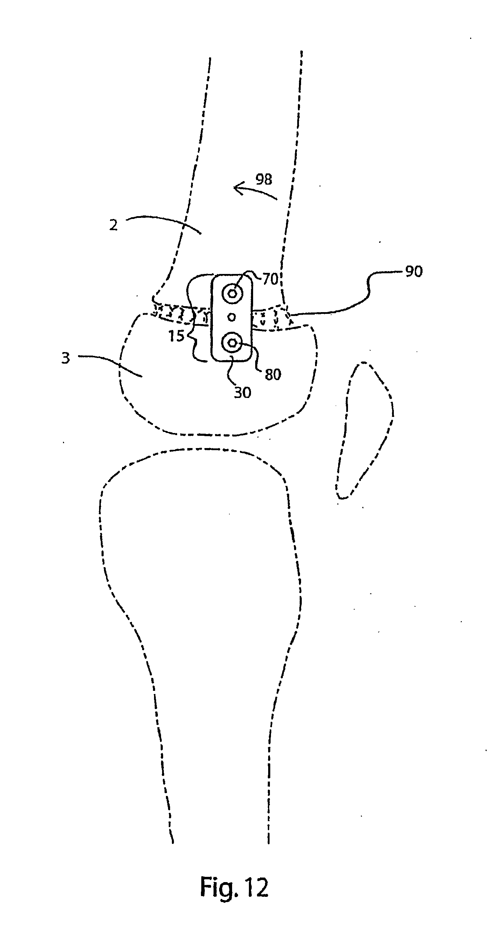

FIG. 11 and FIG. 12 show an example of using the bone alignment implant to correct a torsional abnormality between the diaphyseal section 2 and the metaphyseal section 3. The link 30 is placed across the physis 1 at an angle 18 that is related to the amount of torsional deformity between the bone sections 2 and 3. As the physis 1 generates new physeal tissue 90, the bone alignment implant 15 guides the direction of growth of the bone to allow a torsional correction 98 of the bone alignment.

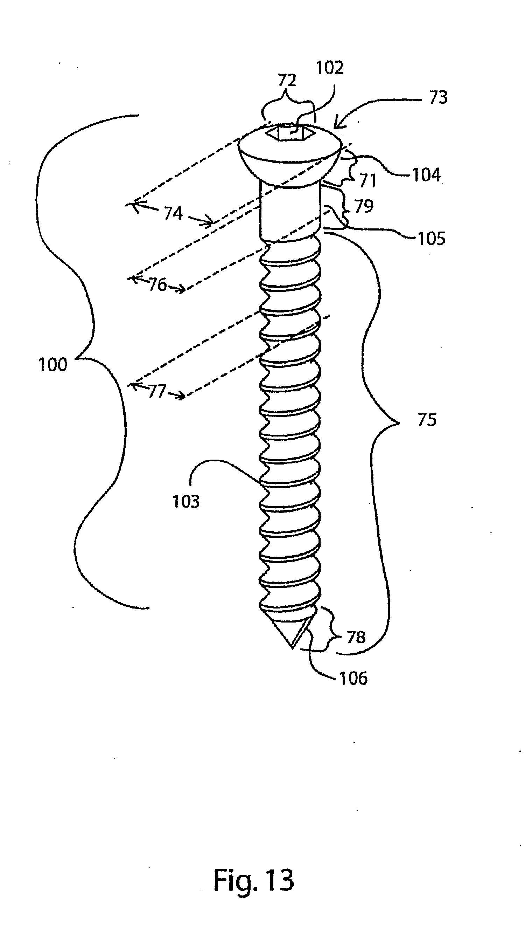

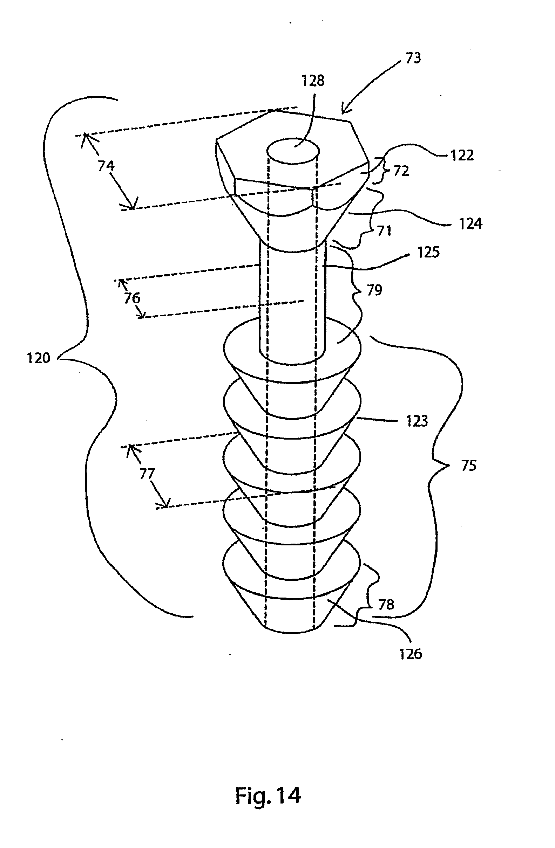

Different fastening devices designs that are well known in the art can be functional as fasteners 70 and 80. The basic common elements of the fasteners 70 and 80 are seen in the example of a threaded fastener 100 in shown in FIG. 13 and a barred fastener 120 shown in FIG. 14.

The threaded fastener 100, and the barbed fastener 120 both have a head 73 comprising a head diameter 74, a drive feature 72 and a head underside 71. The drive feature in the threaded fastener 100 is an internal female hex drive feature 102. The drive feature in the barbed fastener 120 is an external male drive feature 122. The shape of the underside 71 of the barbed fastener 120 is a chamfer cut 124 and the underside of the threaded fastener 100 is rounded cut 104. The underside 71 shape of both the threaded fastener 100 and the barbed fastener 120 examples are dimensioned to mate with shapes of the first opening 31 and the second opening 32 in the link 30.

Directly adjacent to the head 72 on both threaded fastener 100 and the barbed fastener 120 is a fastener shaft 79 with a shaft diameter 76. Protruding from the shaft 79 is the aforementioned engager 75 with a fixation outer diameter 77. This fixation diameter varies depending on the bone that is being treated and the size of the patient. Typically this diameter is from 1 mm to 10 mm. The shaft diameter 76 can be an undercut shaft 125, as shown in the barbed fastener 120, with a diameter 75 smaller than the fixation outer diameter 77. The shaft diameter can also be a run out shaft 105 as shown in the threaded fastener 100 with a diameter 76 larger than or equal to the fixation diameter 77. In either case, the shaft diameter 76 is smaller than the head diameter 74. This allows fasteners 70 and 80 to be captured and not pass completely through the openings 31 and 32 in the link 30.

In the case of the threaded fastener 100, the engager 75 comprises at least one helical thread form 103. Although the example of a unitary continuous helical thread 103 is shown, it is understood that multiple lead helical threads, discontinuous helical threads, variable pitch helical threads, variable outside diameter helical threads, thread-forming self-tapping, thread-cutting self-tapping, and variable root diameter helical threads can be interchanged and combined to form an optimized engager 75 on the threaded fastener 100. The engager 75 on the barbed fastener 120 is shown as a uniform pattern of connected truncated conical sections 123. However, it is understood that different barbed fastener designs known in the art such as superelastic wire arcs, deformable barbs, radially expandable barbs, and barbs with non-circular cross-sections can be interchanged and combined to form a optimized engager 75 on the barbed fastener 120.

Protruding from the engager 75 at the distal end of both the threaded fastener 100 and the barbed fastener 120 is a fastener tip 78. The fastener tip 78 can either be a smooth conical tip 126 as shown in the barbed fastener 120, or a cutting tip 106 as shown on the threaded fastener 100. Although a cutting flute tip is shown as the cutting tip 106 on the threaded fastener, other cutting tips designs including gimble and spade tips can be used.

In the example of the barbed fastener 120, a cannulation bore 128 passes though the head 71, the shaft 79, the engager 75, and the tip 78. This cannulation bore 128 allows placement of the fasters 70 and 80 over the guide pins 40 and 50. Although not shown on the example of the threaded fastener 100 in FIG. 13, it is understood that the fasteners 70 and 80, regardless of their other features, can either be of the cannulated design shown in the barbed fastener 120 example or a non-cannulated design as shown in the threaded fastener 100 example.

Fasteners 70 and 80 can be made in a variety of different ways using a variety of one or more different materials. By way of example and not by limitation, Fasteners 70 and 80 can be made from medical grade biodegradable or non-biodegradable materials. Examples of biodegradable materials include biodegradable ceramics, biological materials, such as bone or collagen, and homopolymers and copolymers of lactide, glycolide, trimethylene carbonate, caprolactone, and p-dioxanone and blends or other combinations thereof and equivalents thereof. Examples of non-biodegradable materials include metals such as stainless steel, titanium, Nitinol, cobalt, alloys thereof, and equivalents thereof and polymeric materials such as non-biodegradable polyesters, polyamides, polyolefins, polyurethanes, and polyacetals and equivalents thereof.

All the design elements of the threaded fastener 100 and barbed fastener 120 are interchangeable. Hence either of the fasteners 70 and 80 can comprise of any combination of the design elements described for the threaded fastener 100 and the barbed fastener 120. By way of one example, the first fastener 70 can be made from a bioabsorbable copolymer of lactide and glycolide and structurally comprise an external male drive feature 122, a run out shaft 105, a multiple-lead, non-continuous helically threaded engager 75, with a cutting flute tip 106 and a continuous cannulation 128. Likewise the second fastener 80 can be made from a different combination of the features used to describe the threaded fastener 100 and the barbed fastener 120.

Although the examples of barbed connected truncated conical sections 123 and helical thread forms 103 are shown by example to represent the bone engager 75, it is understood that other means of engaging bone can be used for the engager 75. These means include nails, radially expanding anchors, pressfits, tapers, hooks, surfaces textured for biological ingrowth, adhesives, glues, cements, hydroxyapitite coated engagers, calcium phosphate coated engagers, and engagers with tissue engineered biological interfaces. Such means are known in the art and can be used as alternative bone engagement means for the first bone engager 75 on the first fastener 70 or the second bone engager 85 on the second fastener 80.

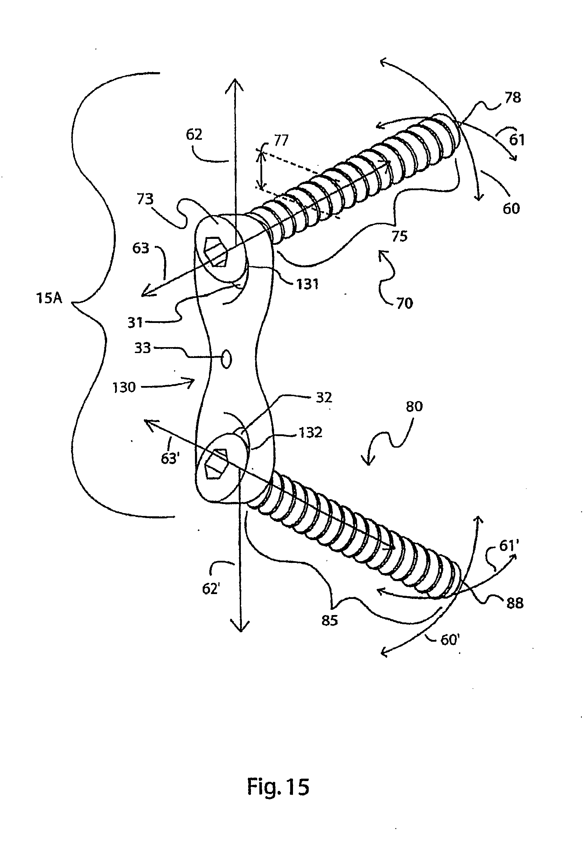

Different embodiments of the bone alignment implant 15 invention allow for different means of relative movement between the two bone sections 2 and 3. Nine embodiments of the bone alignment implant 15 are shown in FIG. 15 through FIG. 23. These embodiments are labelled 15A through 151.

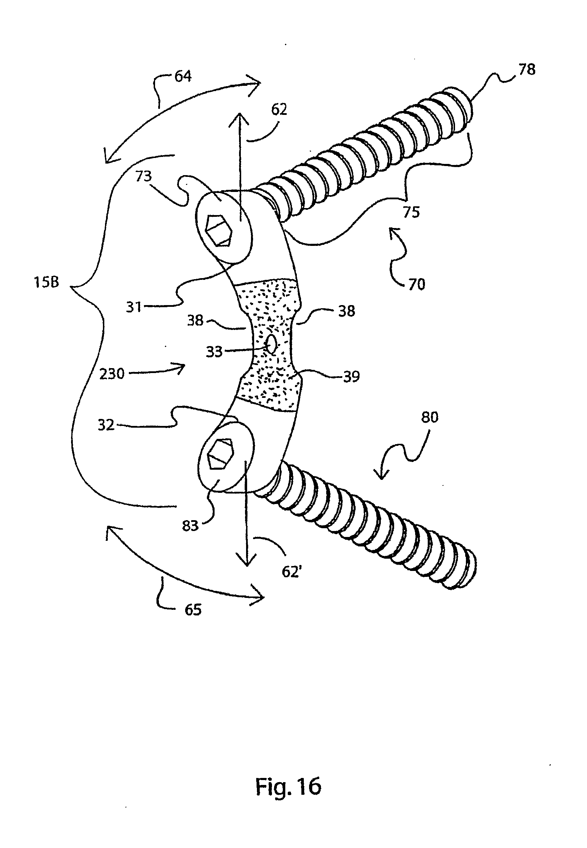

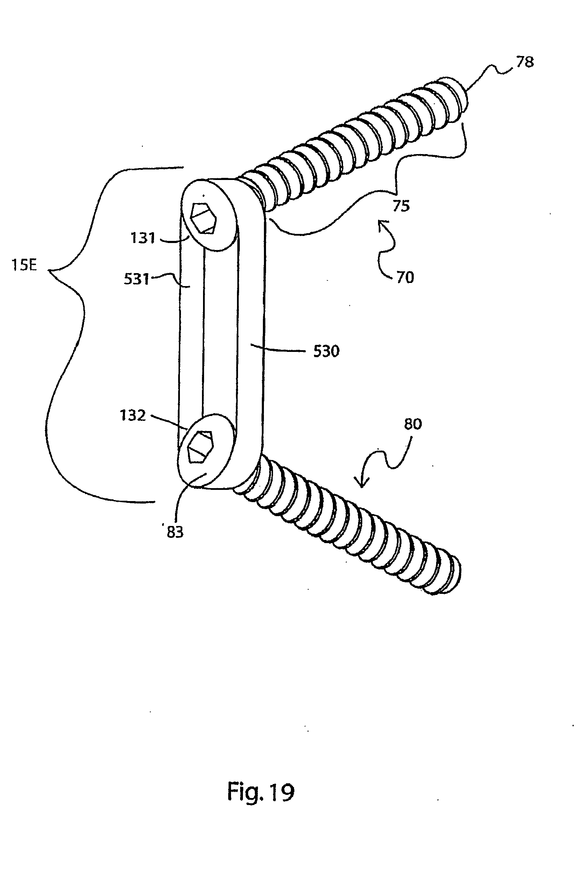

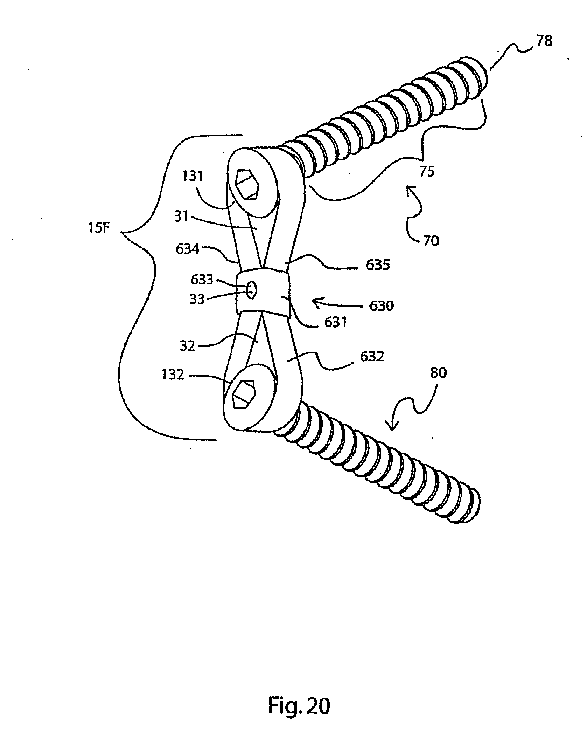

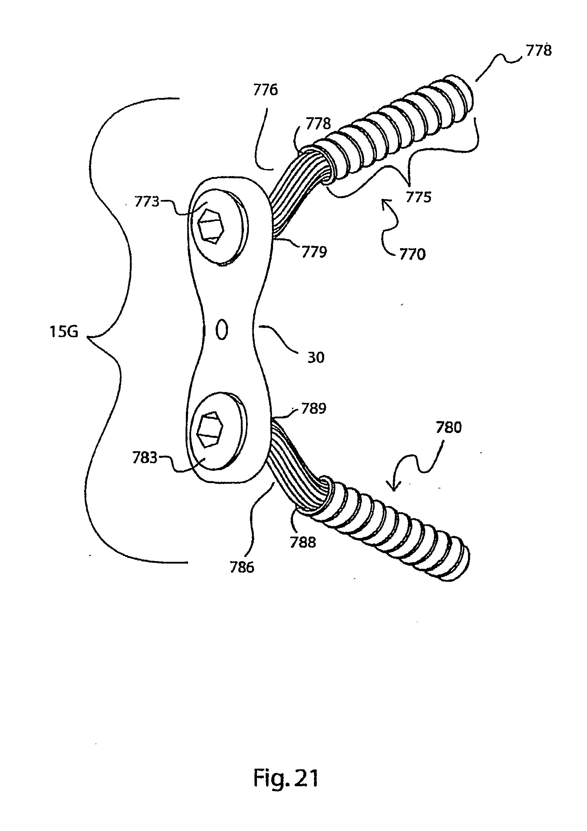

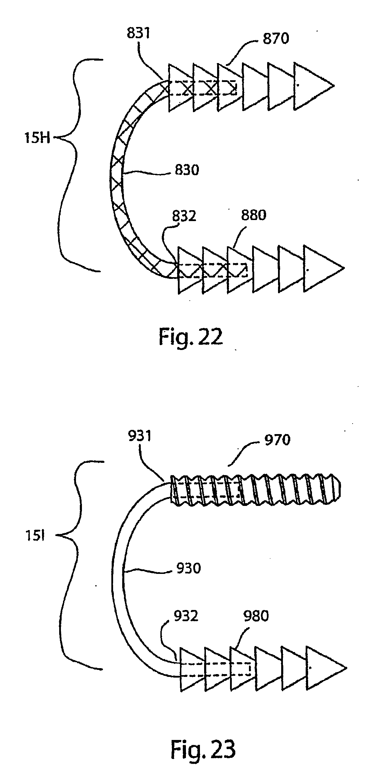

In a rigid-bodies embodiment 15A shown in FIG. 15, both the link 30 and the fasteners 70 and 80 are rigid, but a first connection 131 and a second connection 132 between each of them allows for relative movement between the link 30 and the fasteners 70 and 80 resulting in relative movement between the bone sections 2 and 3. In embodiments 15B, 15C, and 15D of this invention shown in FIG. 16, FIG. 17 and FIG. 18, the link 30 is deformable allowing the fasteners 70 and 80 to move with the bone sections 2 and 3. In embodiments 15E and 15F shown in FIG. 19 and FIG. 20, the connections between the link 30 and the fasteners 70 and 80 along with the deformable link 30 allow the fasteners 70 and 80 to move with the bone sections 2 and 3. In an embodiment 15G shown in FIG. 21, the fasteners 70 and 80 are deformable allowing movement of the bone sections 2 and 3. In embodiments 15H and 151 shown in FIG. 22 and FIG. 23, the fasteners 70 and 80 are fixed to a flexible link 30.

A rigid-bodies embodiment 15A of the bone alignment implant 15 is shown in FIG. 15. In the rigid-bodies embodiment 15A, the link 30 is a rigid link 130. In the rigid bodies embodiment 15A, the first fastener 70 is free to rotate about its axis or tilt in a first tilt direction 60 or a second tilt direction 61 and is partially constrained to move in a longitudinal direction 62 by the confines of the size of the first opening 31 and the first shaft diameter 77, and partially constrained to move in the axial direction by the confines of the size of the first opening and the diameter 74 of the head 73 of the first fastener 70. The first opening 31 is larger in the longitudinal direction 62 than is the shaft diameter 77 of the first fastener 70. This allows for relative movement at the first joint 131 in a combination of tilt in the first direction 60, tilt in the second direction 61, and translation in the axial direction 63.

Similar tilt and translation is achieved between the second fastener 80 and the link 30 at the second joint 132. The second fastener 80 is also free to rotate or tilt in a first tilt direction 60' or a second tilt direction 61' and is partially constrained to move in a longitudinal direction 62' by the confines of the size of the second opening 32 and the shaft diameter of the second fastener 80. The second opening 31 is larger in the longitudinal direction 62' than is the shaft diameter of the second fastener 70. This allows for relative movement at the second joint 132 in a combination of tilt in the first direction 60' and tilt in the second direction 61' and limited translation in the axial direction 63'.

The combination of relative movement between the first joint and the second joint allows for relative movement between the bone sections 2 and 3 when the rigid bodies embodiment 15A of the bone alignment implant 15 is clinically applied across an active physis 1.

A flexible link embodiment 15B of the bone alignment implant 15 is shown in FIG. 16. In the deformable link embodiment 15B, the link 30 is represented by a deformable link 230 that allows deformation of the section 2 and 4 as the physis 1 grows in a first bending direction 64 and a second bending direction 65. However, the maximum length between the first opening 31 and the second opening 32 of the deformable link 230 limits the longitudinal displacement 62 between the head 73 of the first fastener 70 and the longitudinal displacement 62' between the head 83 of the second fastener 80. Since the heads 73 and 83 are coupled to the respective bone engagers 75 and 85, and the bone engagers 75 and 85 are implanted into the respective bone segments 2 and 3, the maximum longitudinal displacement of the bone segments 2 and 3 is limited by the deformed length between the first opening 31 and second opening 32 of the link 30, and the flexibility and length of the fasteners 70 and 80.

Also shown in FIG. 16 is a material differential area 38 on the link 30. The material differential area 38 is an area on the link 30 where material is either added to the link 30 or removed from the link 30 in relationship to the desired mechanical properties of a central section 39 of the link 30. The central section 39 is made stiffer by adding material to the material differential area 38.

The central section 39 is made more flexible by removing material from the material differential area 38. Similarly the central section 39 is made stiffer by holding all other variables constant and decreasing the size of the guide opening 33. The central section 39 is made more flexible by increasing the size of the guide opening 33. Hence the desired stiffness or flexibility of the link 30 is regulated by the relative size of the material removed or added at the material differential areas 37 and 38 and the relative size of the guide opening 33 with respect to the outer periphery 34 in the central section 39 of the link 30.

It is also understood that the relative stiffness and strength of the link 30 and structural elements such as the central section 39 is dependent on the material from which it is made. The link 30 and structural elements such as the central section 39 therein can be made in a variety of different ways using one or more of a variety of different materials. By way of example and not by limitation, the central section 39 can be made from medical grade biodegradable or non-biodegradable materials. Examples of biodegradable materials include biodegradable ceramics, biological materials, such as bone or collagen, and homopolymers and copolymers of lactide, glycolide, trimethylene carbonate, caprolactone, and p-dioxanone and blends or other combinations thereof and equivalents thereof. Examples of non-biodegradable materials include metals such as titanium alloys, zirconium alloys, cobalt chromium alloys, stainless steel alloys, Nitinol alloys, or combinations thereof, and equivalents thereof and polymeric materials such as non-biodegradable polyesters, polyamides, polyolefins, polyurethanes, and polyacetals and equivalents thereof.

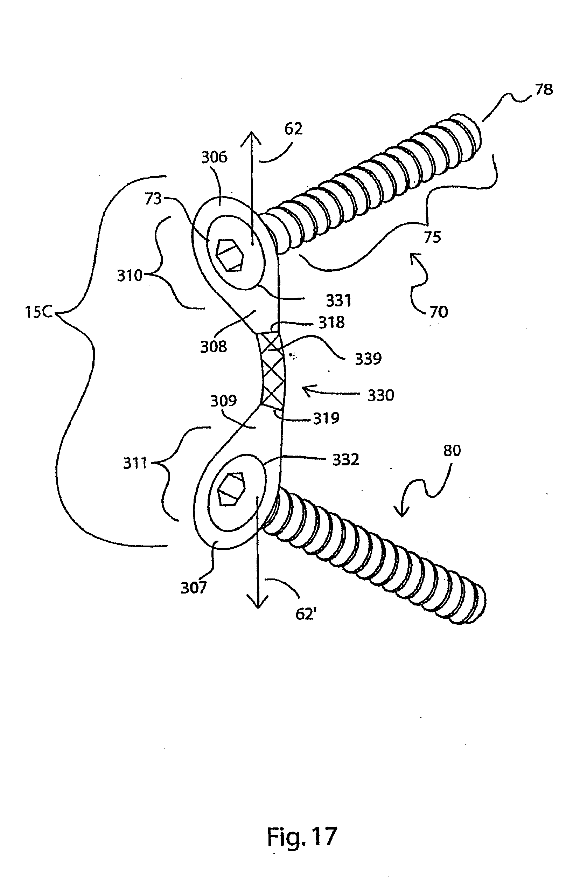

FIG. 17 shows a flexible cable embodiment 15C of the bone alignment implant 15. The flexible cable embodiment 15C comprises a flexible cable link 330 joined to the first fastener 70 by a first eyelet 306 on the first side 310 and joined to the second link 80 by a second eyelet 307 on the second side 311. The first eyelet 306 has a first opening 331 through which the first fastener 70 passes. The second eyelet 307 has a second opening 332 through which the second fastener 80 passes. A flexible member 339 connects the first eyelet 306 to the second eyelet 307. The flexible member 339 allows relative movement between the first eyelet 306 and the second eyelet 307, except the longitudinal displacement 62 and 62' is limited by the length between the first opening 331 and the second opening 332. This is proportional to the length of the flexible member 339.

The flexible member 339 is connected to the first eyelet 306 and the second eyelet 307 by means of joined connections 318 and 319. These joined connections 318 and 319 are shown as crimped connections in this example. However, the flexible member 339 can be joined to the link 30 by other means such as insert molding, welding, soldering, penning, pressfitting, cementing, threading, or gluing them together.

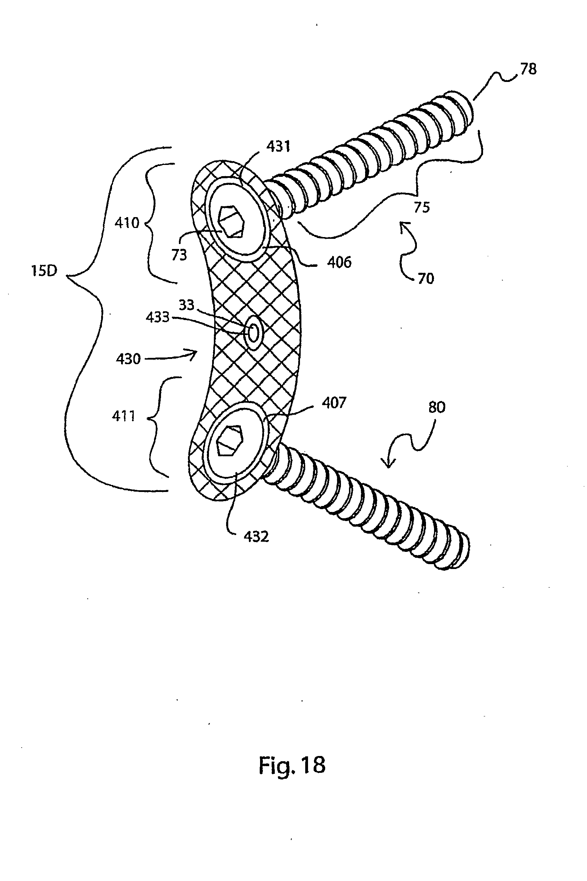

FIG. 18 shows a flexible fabric embodiment 15D of the bone alignment implant 15. The flexible fabric embodiment 15D comprises a flexible fabric link 430 joined to the first fastener and the second fastener 80. The flexible fabric link 430 comprises a first grommet 406 on a first side 410 and joined to the second link 80 by a second grommet 407 on a second side 411. The first grommet 406 has a first opening 431 through which the first fastener 70 passes. The second grommet 407 has a second opening 432 through which the second fastener 80 passes. A flexible fabric 439 connects the first grommet 406 to the second grommet 407. The flexible fabric 439 allows relative movement between the first grommet 406 and the second grommet 407, except the longitudinal displacement 62 is limited by the length between the first opening 431 and the second opening 432. A guide hole grommet 433 may be employed to reinforce the guide pin opening 33.

The grommets function as reinforcement structures that prevent the flexible fabric from being damaged by the fasteners 70 and 80. The grommets can be made from medical grade biodegradable or non-biodegradable materials. Examples of materials from which the grommet can be made are similar to those bioabsorbable and non-biodegradable materials listed as possible materials for the fasteners 70 and 80.

The flexible fabric 439 comprises woven or matted fibers of spun medical grade biodegradable or non-biodegradable materials. A wide variety of materials may be used to make the flexible fabric 439. For example, wire, fibers, filaments and yarns made therefrom may be woven, knitted or matted into fabrics. In addition, even nonwoven structures, such as felts or similar materials, may be employed. Thus, for instance, nonabsorbable fabric made from synthetic biocompatible nonabsorbable polymer yarns, made from polytetrafluorethylenes, polyesters, nylons, polyamides, polyolefins, polyurethanes, polyacetals and acrylic yarns, may be conveniently employed. Similarly absorbable fabric made from absorbable polymers such as homopolymers and copolymers of lactide, glycolide, trimethylene carbonate, caprolactone, and p-dioxanone and blends or other combinations thereof and equivalents thereof may be employed. Examples of non-biodegradable non-polymeric materials from which the flexible fabric can be made include metals such as stainless steel, titanium, Nitinol, cobalt, alloys thereof, and equivalents thereof.

A band embodiment 15E is shown in FIG. 19 in which a band 530 that is a continuous loop or band of material that functions as the link 30. The band embodiment 15E allows both movement at the first joint 131 and second joint 132 and allows deformation within the link 30. The shafts 79 of the first fastener 70 and second fastener 80 are both positioned in the inside 531 of the band 530. The band can be either a fabric band made from the same materials described for the flexible fabric 439 of the flexible fabric embodiment 15D, or the band 530 can be a unitary, continuous loop of a given biocompatible material such as a bioabsorbable polymer, nonbiodegradable polymer, metal, ceramic, composite, glass, or biologic material.

In the band embodiment 15E, the band 530 tethers between the head 73 of the first fastener 70 and the head 83 of the second fastener as the physeal tissue 90 generates and the bone in aligned. One advantage of the band embodiment 15E is that after the desired alignment is obtained, the band 530 can be cut and removed without removing the fasteners 70 and 80. Furthermore, as with all of the embodiments of the bone alignment device 15A, 15B, 15C, 15D, 15F, 15G, 15H and 15I, the fasteners 70 and 80 can be made from a biodegradable material and left in place to degrade.

A crimped band embodiment 15F of the bone alignment device 15 is shown in FIG. 20. The crimped band embodiment 15F is similar to the band embodiment 15E in that it allows both movement at the first joint 131 and second joint 132. The crimped band embodiment 15F comprises a crimped band link 630 that comprises a band 632 that loops around the head 73 of the first fastener 70 and the head 83 of the second fastener 80. However, the link 30 in the crimped band embodiment 15F has an additional ferrule feature 631 comprising a loop of deformable material that brings a first side 634 and a second side 635 of the band together forming the first opening 32 and the second opening 32. A bore 633 in the midsection of the ferrule 631 passes through the crimped band link 630 to form the aforementioned guide pin hole 33.

As with the band embodiment 15E, an advantage of the band embodiment 15E is that after the desired alignment is obtained, the band 632 can be severed across the boundaries of the first opening 31 and the boundaries of the second opening 32. This provides a means for the crimped band link 630 to be removed without removing the fasteners 70 and 80.

A deformable fastener embodiment 15G is shown in FIG. 21. The deformable fastener shaft embodiment 15G comprises a first deformable fastener 770 with a deformable shaft 776, a link 30 and a second fastener 80. The second fastener 80 may also have a deformable shaft 786 as shown in the deformable fastener embodiment 15G. However, it may also have a nondeformable shaft. The second fastener 80 may also be in the design or material of any of the combinations of aforementioned threaded fasters 100 or barbed fasteners 120. Likewise, the second fastener 80 can have a flexible shaft 786, as shown in the example of the deformable fastener embodiment 15G in FIG. 21, and the first fastener 70 can be in the design or material of any of the combinations of aforementioned threaded fasters 100 or barbed fasteners 120.

The flexibility of the flexible shaft 776 and 786 of the fasteners 70 and 80 can be simply a result of the material selection of the flexible shaft 776 and 786, or can be the result of a design that allows for flexibility of the shaft. For example, the flexible shaft 776 and 786 can be manufactured from a material such as the aforementioned biocompatible polymeric materials or superelastic metallic materials such as Nitinol that would deform under the loads associated with bone alignment. The flexible shafts 776 and 786 could also be manufactured from biocompatible materials typically not considered to be highly elastic such as stainless steel, titanium, zirconium, cobalt chrome and associated alloys thereof, and shaped in the form of a flexible member such as cable, suture, mesh, fabric, braided multifilament strand, circumferentially grooved flexible shaft, filament, and yarn.

Connections 778 and 788 between the flexible shafts 776 and 786 and the associated engagers 775 and 780 of the fasteners 70 and 80 can be unitary and continuous, as is typically the case for fasteners 70 and 80 made entirely from the aforementioned biocompatible polymeric materials and superelastic metallic materials. The connections 778 and 7,88 can also be joined connections as is the case for flexible shafts 776 and 786 made from flexible members. Although the example of a pressfit connection is shown as the means of the connections 778 and 788 in the deformable fastener embodiment 15G shown in FIG. 21, these joined connections 778 and 788 can be crimped, welded, insert molded, soldered, penned, pressfit, cemented, threaded, or glued together.

Heads 773 and 783 are connected to the respective flexible shafts 776 and 786 by respective head connections 779 and 789. These head connections 779 and 789 can also be unitary and continuous, as again is typically the case of fasteners 70 and 80 made entirely from the aforementioned biocompatible polymeric materials and superelastic metallic materials. The head connections 779 and 789 can also be joined connections, as is the case for flexible shafts 776 and 786 made from flexible members. Although the example of a pressfit connection is the means of the connections 779 and 789 in the deformable fastener embodiment 15G shown in FIG. 21, these joined connections 779 and 789 can also be crimped, insert molded, welded, soldered, penned, pressfit, cemented, threaded, or glued together.

Embodiments of the bone alignment implant 15 are shown in FIG. 22 and 23 in which the first fastener 70 and second fastener 80 are fixedly joined to the link 30 that is flexible.

A paired fastener embodiment 15H is shown in FIG. 22 in which similar designs of paired fasteners 870 and 880 are fixedly joined to a flexible link 830 by means of joined connections 831 and 832. These joined connections 831 and 832 are shown as insert molded connections in this example in which the link is formed within the fastener by means of molding the molded fasteners 870 and 880 around the flexible link 830. However, the pair fasteners 870 and 880 can be joined to the link 830 by other means such as crimping, welding, soldering, penning, pressfitting, cementing, threading, or gluing.

In the paired fastener embodiment 15H, the first paired fastener 870 and the second paired fastener 880 are shown in FIG. 22 as barbed style fasteners similar to the aforementioned barbed fastener 120. However, the paired fasteners 870 and 880 can also be similar to the aforementioned threaded fastener 100 or can comprise of any combination of the design elements described for the threaded fastener 100 and the barbed fastener 120.

A non-paired fastener embodiment 151 is shown in FIG. 23 in which different designs of fasteners 970 and 980 are fixedly joined to a flexible link 930 by means of joined connections 931 and 932. These joined connections 931 and 932 are shown as insert molded connections in this example in which the link is formed within the fastener by means of molding the molded fasteners 970 and 980 around the flexible link 930. However, the fasteners 970 and 980 can be joined to the link by other means such as crimping, welding, soldering, penning, pressfitting, cementing, threading, or gluing.

Spinal deformities, such as scoliosis, may be treated using the bone alignment implant of the invention.

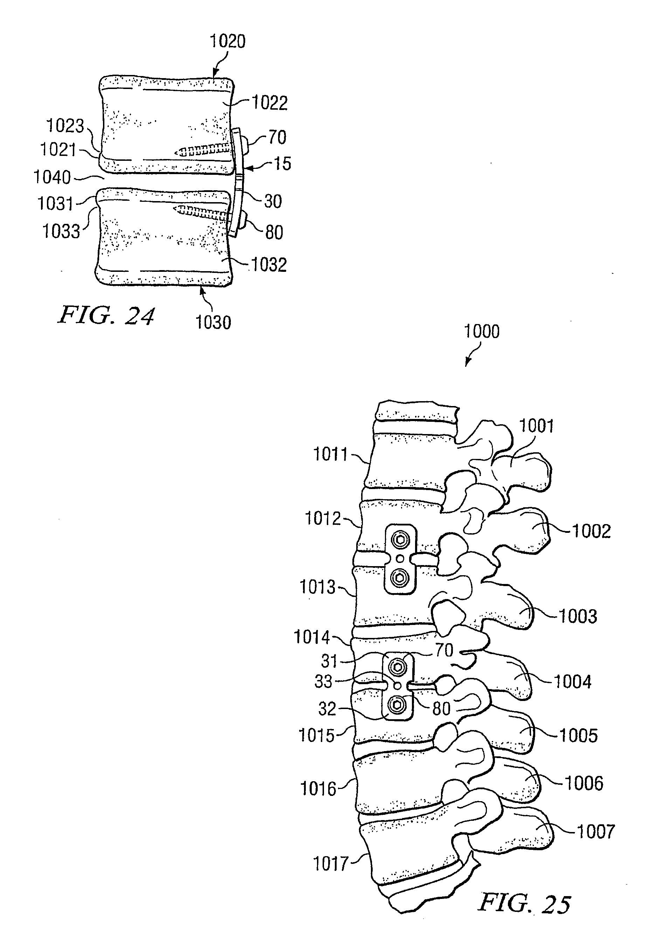

Referring first to Fig. 24, an anterior view of two vertebral bodies, 1020 and 1030, connected by a bone implant 15. Vertebral body 1020 has a metaphyseal section 1021 and a diaphyseal section 1022 separated by a physis 1023. Similarly, vertebral body 1030 has a metaphyseal section 1031 and a diaphyseal section 1032 separated by a physis 1033. Vertebral bodies 1020 and 1030 are separated by a intervertebral disc 1040.

Still referring to Fig. 24, a bone alignment implant 15 is shown positioned for correction of a two-dimensional curvature wherein the convex side of the curve is proximal to the bone alignment implant 15. The bone alignment implant 15 comprises the link 30, the first fastener 70, and the second fastener 80. The bone alignment implant 15 functions as a tether connecting the diaphyseal section 1022 of vertebral body 1020 to the diaphyseal section 1032 of vertebral body 1030. As the physis 1023 of vertebral body 1020 and the physis 1033 of vertebral body 1030 generate new physeal tissue, the new physeal tissue will fill in those areas subjected to the least resistance. Referring to Fig. 24, the new physeal tissue generated by physis 1023 will fill in between the diaphyseal section 1022 and the metaphyseal section 1021 distal to the bone alignment implant 15. Likewise, new physeal tissue generated by physis 1033 will fill in between diaphyseal section 1032 and metaphyseal section 1031 distal to the bone alignment implant 15. The bone alignment implant 15 restricts the longitudinal movement between vertebral body 1020 and vertebral body 1030 thereby providing resistance to new physeal tissue proximal to the bone alignment implant 15.

Referring to Fig. 25, a sagittal view of a portion of a spinal column 1000 is shown, having a plurality of vertebra 1001, 1002, 1003, 1004, 1005, 1006, and 1007, and corresponding vertebral bodies, 1011, 1012, 1013, 1014, 1015, 1016, and 1017. As shown in Fig. 25, fusionless correction or improvement of scoliosis may be obtained by implanting one or more bone alignment implants 15 across two or more of the vertebrae in a convex region of a spinal curvature. As shown in Fig. 25, bone alignment implants are placed spanning vertebrae 1002 and 1003; 1004 and 1005. A first fastener 70 is placed through a first opening 31 and is attached within a diaphyseal section of vertebral body 1014 and a second fastener 80 is placed through a second opening 32 and attached within a diaphyseal section of vertebral body 1015.

In a procedure similar to that shown in Figs. 1 and 3, the bone alignment implant may be properly located for spinal applications using a guide wire 8 and/or guide 20. However, because the bone alignment implant 15 is placed across intervertebrally, rather than intravertebrally, the dimensions of the guide and relative distances between a first and second guide pin would be appropriately adjusted.

While Figs. 24 and 25 refer to use of bone alignment implant 15, it will be understood that any of the embodiments of the bone alignment implant discussed herein may be used in the treatment of spinal deformities.

Moreover, while FIGS. 24 and 25 are discussed in connection with attaching the bone alignment implant on the convex side of a spinal deformities, in other aspects of the invention, the bone alignment implant may be attached on the concave side of a spinal deformity. For example, in alternative embodiments of the bone alignment implant, a link portion of the bone alignment implant may be formed from a substantially stiff or expandable material such that an expansion force is exerted on the concave portion of the spinal deformity.

The bone alignment implants may be used in any of the spinal regions, including the cervical spine, the thoracic spine, lumbar or sacrum spine. Moreover, while illustrated in Figs. 24 and 25 in positions to correct medial-lateral curvature, the bone implant may be used to treat anterior-posterior and cranial-caudal curvatures as well. In addition, in some embodiments, the bone implant may be used to treat spinal rotation deformities in addition to or apart from two-dimensional curvatures.

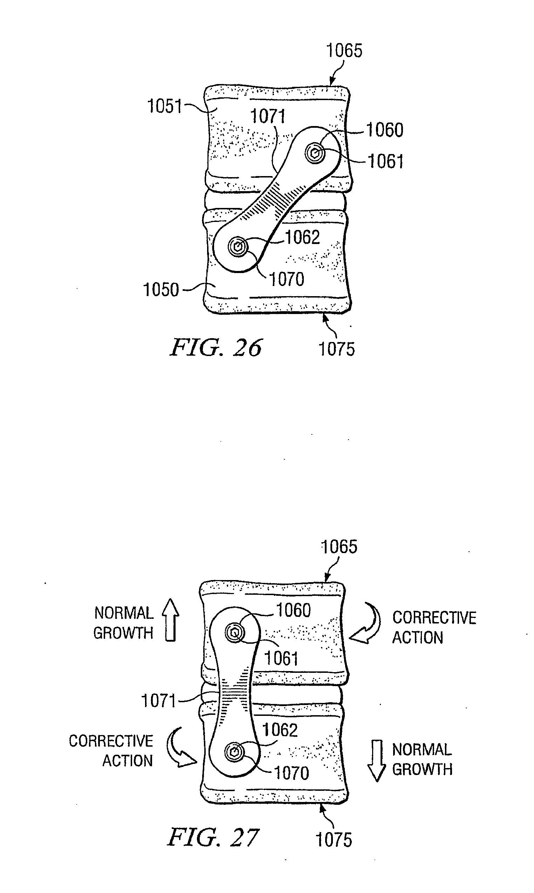

For example, use of an embodiment of the bone alignment implant is shown in use to correct a rotation deformity is illustrated in Figs. 26 and 27. As can be seen in FIG. 26, a first fastener 1061 passes through a first opening 1060 and is attached to the vertebral body 1051 of a first vertebra 1065. A second fastener 1062 passes through a second opening 1070 and is attached to the vertebral body 1050 of a second vertebra 1075. As can be seen in FIG. 26, the attachment of the first fastener 1061 is axially displaced from that of the second fastener 1062. Referring now to FIG. 27, it is shown that as normal longitudinal growth of the vertebra occurs, the bone alignment implant applies a rotational force on the first and second vertebra 1065 and 1075 so as to correct the rotational deformity. In preferred embodiments of the invention, fastener 1061 and 1062 are permitted to rotate with first openings 1060 and 1070, respectively. In alternative embodiments, only one of the fastener 1061 or 1062 is permitted to rotate substantially within the appropriate opening, 1060 or 1070. In those applications in which the bone alignment implant is used to correct rotational deformities, the bone alignment implant preferably includes a substantially rigid link 1071. As shown in both FIGS. 26 and 27, the link 1071 of the bone alignment implant does not include a guide opening.

Bone alignment implants having a link portion of sufficient size to span across more than two vertebra may be used. The link portion of the bone alignment implant may be expandable so as to provide an adjustable distance between first and second openings in the bone alignment implant. Any of a number of currently known or later developed configurations to provide such an expandable link portion, such as a folded portion, may be employed.

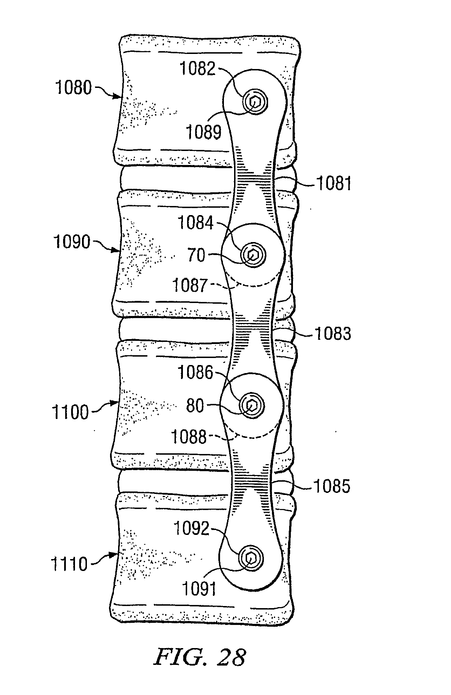

Two or more bone alignment implants may be linked together to form a chain of bone alignment implants attached across three or more vertebra. As shown in Fig. 28, four vertebrae 1080, 1090, 1100, 1110 are attached using three partially overlapping bone alignment implants 1081, 1083, and 1085. A first fastener 70 passes through a second opening of first bone alignment 1081 and a first opening 1084 of second bone alignment implant 1083. A second fastener 80 passes through a second opening of second bone alignment implant 1083 and a first opening 1086 of third bone alignment implant 1085. As used herein the term "chain of bone alignment implants" means two or more bone alignment implants partially overlapping and sharing a fastener as depicted in Fig. 28. Referring again to Fig. 28, dashed line 1087 depicts that portion of the first bone alignment implant 1081 which lies under second bone alignment implant 1083. Similarly, dashed line 1088 depicts that portion of second bone alignment implant 1083 which lies under third bone alignment implant 1085. Fig. 28 depicts a chain of only 3 bone alignment implants. Thus, the first bone alignment implant 1081 is fastened to first vertebra 1080 by passing a fastener 1089 through a first opening 1082. Similarly, the third bone alignment implant 1085 is fastened to vertebra 1110 by passing fastener 1091 through a second opening 1092. It will be understood that the number of bone fasteners used in such a chain of bone alignment implants may be decreased or increased according to the type, location and severity of the spinal deformity. Moreover, a combination of vertical or substantially vertical placement and angled placement along one or more chains of bone alignment implants may be used in a single patient to correct both spinal curvature and rotational deformities.

Implantation of the bone alignment implant for correction of a deformity in the thoracic spine may be carried out by an anterior thoracoscopic procedure which is minimally invasive. Thorascopic spinal surgical methods are known and may be adapted for use in implanting the bone alignment implant of the invention. Open procedures, wherein a relatively long incision is used to expose the vertebrae to which the implant(s) is(are) to be connected may also be used. Alternatively, implantation for deformities in any of the spinal regions may occur by way of posterior procedure or some combination of anterior and posterior procedures. Where the bone alignment implant is attached intervertebrally, a guide wire is generally not used. In such cases, the location of bone engagers placement may be determined by visual or other inspection. For example a k-wire or pin may be used to locate the center of the vertebral body of each vertebra involved. Following location of the center of the vertebral body, a cannulated drill may be placed over the k-wire and a hole for the bone engager drilled. Once the hole is drilled, the k-wire may be placed through the appropriate bone engager opening and the bone alignment implant slid down the k-wire. The bone engager is then screwed into the hole.

The bone alignment implant may be used intravertebrally wherein a k-wire may be used to locate the physis and the bone alignment implant attached to the diaphyseal and metaphyseal sections of a single vertebral body.

Use of the bone alignment implant permits whole or partial correction of the spinal curvature while permitting some growth along the length of the spine on the concave side of the deformity. Thus, overall loss of body height in patients having the invention is usually not severe, and most frequently, not as severe as that in patients in which fusion techniques are utilized.

The bone alignment implants may be removed, preferably following complete or partial correction of the spinal deformity, thereby permitting continued vertebral growth and attainment of height.

While the present invention has been disclosed in its preferred form, the specific embodiments thereof as disclosed and illustrated herein are not to be considered in a limiting sense as numerous variations are possible. The invention may be embodied in other specific forms without departing from its essential characteristics. The described embodiments are to be considered in all respects only as illustrative and not restrictive. The scope of the invention is, therefore, indicated by the appended claims rather than by the foregoing description.

## Claims

1. A bone alignment implant for correcting spinal deformities, comprising:
a first fastener with a first engager adapted for connection to a first vertebral body;
a second fastener with a second engager adapted for connection to a second vertebral body;
a third fastener with a third engager adapted for connection to a third vertebral body; and
first and second links connecting the first, second, and third fasteners;
wherein the first and second links are aligned in an orientation in which an opening of the first link overlaps an opening of the second link;
wherein the first fastener passes through both overlapping openings in said first and second links;
wherein the first and second links each have an additional opening axially displaced from said overlapping openings and defined therethrough for receiving either the second fastener or the third fastener; and
wherein each of the first and second links and the fasteners connected thereto are operable to function together as a flexible tether among first, second, and third vertebral bodies

2. A bone alignment implant as in claim 1, wherein each of the first and second link is adapted to span between one or more intervertebral spaces.

3. A bone alignment implant as in claim 1, wherein the first or second link is a flexible link.

4. A bone alignment implant as in claim 1, wherein first or second link is a rigid link.

5. A bone alignment implant as in claim 1, wherein the first engager is a threaded engager.

6. A bone alignment implant as in claim 5, wherein the second engager is a threaded engager.

7. A bone alignment implant as in claim 1, wherein the first fastener of further has a first head, and a first shaft, and the second fastener further has a second head and a second shaft.

8. A bone alignment implant as in claim 7, wherein the first shaft is a deformable shaft.

9. A bone alignment implant as in claim 8, wherein the second shaft is a deformable shaft.

10. A bone alignment implant as in claim 9, wherein the first engager is a barbed engager.

11. A bone alignment implant as in claim 9, wherein the second engager is a barbed engager.

12. A bone alignment implant as in claim 1, wherein the first fastener comprises a head having a rounded-cut underside, and wherein the underside shape is dimensioned to mate with the shape of the overlapping opening in the first link.

## Patentansprüche

1. Ein Knochenausrichtungsimplantat zur Korrektur von Wirbelsäulendeformationen bestehend aus:
einem ersten Befestigungselement, das zur Befestigung an einem ersten Wirbelkörper adaptiert wurde;
einem zweiten Befestigungselement, das zur Befestigung an einem zweiten Wirbelkörper adaptiert wurde;
einem dritten Befestigungselement, das zur Befestigung an einem dritten Wirbelkörper adaptiert wurde;
erste und zweite Verbindungsglieder, welche das erste, zweite und dritte Befestigungselement verbinden;
wobei das erste und zweite Verbindungsglied so ausgerichtet sind, dass eine Öffnung des ersten Verbindungsglieds eine Öffnung des zweiten Verbindungsglieds überlagert;
wobei das erste Befestigungselement durch beide sich überlagernden Öffnungen im genannten ersten und zweiten Verbindungsglied verläuft;
wobei das erste und zweite Verbindungsglied jeweils eine zweite Öffnung aufweist, die von den genannten sich überlagernden Öffnungen axial versetzt ist und dadurch definiert wird, dass sie entweder das zweite oder dritte Befestigungselement aufnimmt; und
wobei das erste und zweite Verbindungsglied und die damit verbundenen Befestigungselemente jeweils gemeinsam als flexible Halterung zwischen dem ersten, zweiten und dritten Wirbelkörper fungieren können.

2. Ein Knochenausrichtungsimplantat entsprechend Anspruch 1, wobei das erste und zweite Verbindungsglied jeweils so adaptiert ist, dass es sich über einen oder mehrere Zwischenwirbelräume erstreckt.

3. Ein Knochenausrichtungsimplantat entsprechend Anspruch 1, wobei das erste oder zweite Verbindungsglied ein flexibles Verbindungsglied ist.

4. Ein Knochenausrichtungsimplantat entsprechend Anspruch 1, wobei das erste oder zweite Verbindungsglied ein starres Verbindungsglied ist.

5. Ein Knochenausrichtungsimplantat entsprechend Anspruch 1, wobei das erste eingreifende Element ein Gewindeelement ist.

6. Ein Knochenausrichtungsimplantat entsprechend Anspruch 5, wobei das zweite eingreifende Element ein Gewindeelement ist.

7. Ein Knochenausrichtungsimplantat entsprechend Anspruch 1, wobei das erste Befestigungselement zudem einen ersten Kopf und einen ersten Schaft hat und das zweite Befestigungselement zudem einen zweiten Kopf und einen zweiten Schaft hat.

8. Ein Knochenausrichtungsimplantat entsprechend Anspruch 7, wobei der erste Schaft ein verformbarer Schaft ist.

9. Ein Knochenausrichtungsimplantat entsprechend Anspruch 8, wobei der zweite Schaft ein verformbarer Schaft ist.

10. Ein Knochenausrichtungsimplantat entsprechend Anspruch 9, wobei das erste eingreifende Element mit einem Widerhaken versehen ist.

11. Ein Knochenausrichtungsimplantat entsprechend Anspruch 9, wobei das zweite eingreifende Element mit einem Widerhaken versehen ist.

12. Ein Knochenausrichtungsimplantat entsprechend Anspruch 1, wobei das erste Befestigungselement einen Kopf mit einer abgerundeten Unterseite aufweist und wobei die Form der Unterseite so dimensioniert ist, dass sie sich in die Form der sich überlagernden Öffnung des ersten Verbindungsglieds einpasst.

## Revendications

1. Un implant pour alignement osseux destiné à corriger des malformations de la colonne vertébrale, comprenant :
un premier élément de fixation avec un premier dispositif de mise en prise adapté pour un raccordement à un premier corps vertébral,
un deuxième élément de fixation avec un deuxième dispositif de mise en prise adapté pour un raccordement à un deuxième corps vertébral,
un troisième élément de fixation avec un troisième dispositif de mise en prise adapté pour un raccordement à un troisième corps vertébral, et
un premier et un deuxième éléments de liaison raccordant les premier, deuxième et troisième éléments de fixation,
où les premier et deuxième éléments de liaison sont alignés dans une orientation dans laquelle une ouverture du premier élément de liaison chevauche une ouverture du deuxième élément de liaison,
où le premier élément de fixation passe au travers des deux ouvertures en chevauchement dans lesdits premier et deuxième éléments de liaison,
où les premier et deuxième éléments de liaison possèdent chacun une ouverture additionnelle axialement décalée desdites ouvertures en chevauchement et définie au travers de celles-ci de façon à recevoir soit le deuxième élément de fixation ou le troisième élément de fixation, et
où chacun des premier et deuxième éléments de liaison et les éléments de fixation raccordés à ceux-ci sont conçus de façon à fonctionner conjointement sous la forme d'une attache flexible entre les premier, deuxième et troisième corps vertébraux.

2. Un implant pour alignement osseux selon la Revendication 1, où chacun des premier et deuxième éléments de liaison est adapté de façon à s'étendre entre un ou plusieurs espaces intervertébraux.

3. Un implant pour alignement osseux selon la Revendication 1, où le premier ou le deuxième élément de liaison est un élément de liaison flexible.

4. Un implant pour alignement osseux selon la Revendication 1, où le premier ou le deuxième élément de liaison est un élément de liaison rigide.

5. Un implant pour alignement osseux selon la Revendication 1, où le premier dispositif de mise en prise est un dispositif de mise en prise fileté.

6. Un implant pour alignement osseux selon la Revendication 5, où le deuxième dispositif de mise en prise est un dispositif de mise en prise fileté.

7. Un implant pour alignement osseux selon la Revendication 1, où le premier élément de fixation possède en outre une première tête et un premier arbre, et le deuxième élément de fixation possède en outre une deuxième tête et un deuxième arbre.

8. Un implant pour alignement osseux selon la Revendication 7, où le premier arbre est un arbre déformable.

9. Un implant pour alignement osseux selon la Revendication 8, où le deuxième arbre est un arbre déformable.

10. Un implant pour alignement osseux selon la Revendication 9, où le premier dispositif de mise en prise est un dispositif de mise en prise à crans.

11. Un implant pour alignement osseux selon la Revendication 9, où le deuxième dispositif de mise en prise est un dispositif de mise en prise à crans.

12. Un implant pour alignement osseux selon la Revendication 1, où le premier élément de fixation comprend une tête possédant une face inférieure à découpe arrondie, et où la forme de la face inférieure est dimensionnée de façon à s'accoupler à la forme de l'ouverture en chevauchement dans le premier élément de liaison.
